(19) Europäisches Patentamt
European Patent Office
Office européen des brevets

(11) **EP 4 629 251 A1**

(12) **EUROPEAN PATENT APPLICATION**
published in accordance with Art. 153(4) EPC

(43) Date of publication:
**08.10.2025 Bulletin 2025/41**

(21) Application number: **23897580.9**

(22) Date of filing: **20.11.2023**

(51) International Patent Classification (IPC):
*G16C 20/70* (2019.01)

(52) Cooperative Patent Classification (CPC):
**G16C 20/70**

(86) International application number:
**PCT/JP2023/041624**

(87) International publication number:
**WO 2024/116932 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2022 JP 2022191909**

(71) Applicant: **Yokogawa Electric Corporation
Tokyo 180-8750 (JP)**

(72) Inventors:
• **MANABE Yuuka**
**Musashino-shi, Tokyo 180-8750 (JP)**
• **OGAWA Jun-ichi**
**Musashino-shi, Tokyo 180-8750 (JP)**
• **IMAMURA Yusuke**
**Musashino-shi, Tokyo 180-8750 (JP)**

(74) Representative: **Grünecker Patent- und
Rechtsanwälte
PartG mbB
Leopoldstraße 4
80802 München (DE)**

(54) **REACTIVITY PREDICTION SYSTEM AND REACTIVITY PREDICTION METHOD**

(57)     A reactivity prediction system (40) includes: a storage unit (43) that stores a learned model (MD) that has learned a relationship between measured reaction parameters and reaction conditions under which the reaction parameters are measured; and a processing unit (44) that predicts reaction parameters from input reaction conditions using the learned model (MD).

FIG. 3

EP 4 629 251 A1

**Description**

TECHNICAL FIELD

The present invention relates to a reactivity prediction system and a reactivity prediction method.

BACKGROUND ART

Patent Document 1 described below discloses an optimal synthesis route development method including: calculating an activation energy of a plurality of synthesis routes of a target compound to be synthesized from data including a structure and an energy of a transition state of a chemical reaction obtained from a quantum chemical calculation; predicting a synthesis yield from data of a yield prediction formula on the synthetic reaction to the plurality of synthesis routes of the target compound to be synthesized; constructing a synthesis route ranking formula from the activation energy and the synthesis yield; and ranking and narrowing down an optimal synthesis route for synthesizing the target compound to propose the optimal synthesis route.

Patent Document 2 described below discloses a synthesis route evaluation system for extracting an optimal synthesis route from a plurality of synthesis routes with respect to a target compound to be synthesized, the system including: an arithmetic operation means including a quantum chemical calculation unit, a reaction mechanism analysis unit, and a synthesis route ranking unit; and a memory means for storing data relating to the synthesis routes.

[Citation List]

[Patent Document]

[Patent Document 1] Japanese Unexamined Patent Application, First Publication No. 2008-150337
[Patent Document 2] Japanese Unexamined Patent Application, First Publication No. 2010-9257

SUMMARY OF INVENTION

Technical Problem

In the above-described related art, the activation free energy ($\Delta G^{\ddagger}$), which is a reaction parameter used as input data for predicting synthesis routes, is obtained from a quantum chemical calculation. Since this $\Delta G^{\ddagger}$ is not a measured value but a value calculated from a simulation, there is a large deviation from the measured value.

The present invention has been made in consideration of the above-described problem, and an object of the present invention is to provide a reactivity prediction system and reactivity prediction method that can predict a reaction parameter with higher accuracy than that in the related art.

Solution to Problem

To solve the above-described problem, a reactivity prediction system according to a first aspect of the present invention includes: a storage unit that stores a learned model that has learned a relationship between measured reaction parameters and reaction conditions under which the reaction parameters are measured; and a processing unit that predicts reaction parameters from input reaction conditions using the learned model.

The reactivity prediction system according to a second aspect of the present invention further includes: a generation unit that generates the learned model using the measured reaction parameters as training data and the reaction conditions under which the reaction parameters are measured as first datasets in the reactivity prediction system according to the first aspect of the present invention.

In the reactivity prediction system according to a third aspect of the present invention, the reaction conditions of the first datasets in the reactivity prediction system according to the second aspect of the present invention include at least one of a reactant, a solvent, a catalyst, an additive, a reagent, a temperature, and a reaction time.

The reactivity prediction system according to a fourth aspect of the present invention further includes: a first interface connectable to a measurement device that measures the reaction parameters in the reactivity prediction system according to any one of the first to third aspects of the present invention.

In the reactivity prediction system according to a fifth aspect of the present invention, the learned model in the reactivity prediction system according to any one of the first to fourth aspects of the present invention is trained to learn a relationship between a feature amount relating to a reaction obtained by a quantum chemical calculation and calculation conditions under which the quantum chemical calculation is performed, as well as the relationship between the measured reaction parameters and the reaction conditions under which the reaction parameters are measured.

The reactivity prediction system according to a sixth aspect of the present invention further includes: a generation unit that generates the learned model using the measured reaction parameters as training data, the reaction conditions under which the reaction parameters are measured as first datasets, and the feature amount and the calculation conditions as a second dataset in the reactivity prediction system according to the fifth aspect of the present invention.

In the reactivity prediction system according to a seventh aspect of the present invention, the feature amount of the second dataset in the reactivity prediction system according to the sixth aspect of the present invention includes at least one of a dipole moment, atomic charge, frequency, and a molecular orbital.

A reactivity prediction system according to an eighth aspect of the present invention further includes: a second interface connectable to a calculation device that performs the quantum chemical calculation in the reactivity prediction system according to any one of the fifth to seventh aspects of the present invention.

The reactivity prediction system according to a ninth aspect of the present invention further includes: a reaction condition presentation unit that presents predetermined reaction conditions based on the reaction parameters predicted by the processing unit in the reactivity prediction system according to any one of the first to eighth aspects of the present invention.

In the reactivity prediction system according to a tenth aspect of the present invention, the reaction condition presentation unit in the reactivity prediction system according to the ninth aspect of the present invention presents reaction conditions expected to have the lowest reaction parameter among the reaction parameters predicted by the processing unit as the predetermined reaction conditions.

The reactivity prediction system according to an eleventh aspect of the present invention further includes: a reaction condition presentation unit that presents predetermined reaction conditions based on the reaction parameters predicted by the processing unit and predetermined constraint conditions in the reactivity prediction system according to any one of the first to tenth aspects.

In the reactivity prediction system according to a twelfth aspect of the present invention, the predetermined constraint conditions in the reactivity prediction system according to the eleventh aspect of the present invention include at least one of a reaction time, a reaction field temperature, and a concentration of a reactant.

In the reactivity prediction system according to a thirteenth aspect of the present invention, the reaction parameters in the reactivity prediction system according to the first aspect of the present invention are thermodynamic parameters.

In the reactivity prediction system according to a fourteenth aspect of the present invention, the thermodynamic parameters in the reactivity prediction system according to the thirteenth aspect of the present invention are activation free energy or reaction enthalpy.

A reactivity prediction method according to a fifteenth aspect of the present invention includes: a first step of inputting reaction conditions; and a second step of predicting reaction parameters from reaction conditions input in the first step using a learned model which has learned a relationship between measured reaction parameters and reaction conditions under which the reaction parameters are measured. Advantageous Effects of Invention

According to one aspect of the present invention described above, reaction parameters can be predicted with higher accuracy than that in the related art.

BRIEF DESCRIPTION OF DRAWINGS

[FIG. 1] A block diagram showing a configuration example of a soft sensor according to a first embodiment of the present invention.

[FIG. 2] A block diagram showing a key configuration of a reactivity prediction system according to the first embodiment of the present invention.

[FIG. 3] A block diagram showing an example of a functional configuration pertaining to generation of a learned model of the reactivity prediction system according to the first embodiment of the present invention.

[FIG. 4] An explanatory view showing an example of learning datasets used in the first embodiment of the present invention.

[FIG. 5] A flowchart showing an example of model generation processing performed by the reactivity prediction system according to the first embodiment of the present invention.

[FIG. 6] A block diagram showing an example of a functional configuration pertaining to $\Delta G^{\ddagger}$ prediction of the reactivity prediction system according to the first embodiment of the present invention.

[FIG. 7] A flowchart showing an example of $\Delta G^{\ddagger}$ prediction processing performed by the reactivity prediction system according to the first embodiment of the present invention.

[FIG. 8] A block diagram showing a key configuration of a reactivity prediction system according to a second embodiment of the present invention.

[FIG. 9] An explanatory view showing an example of learning datasets used in the second embodiment of the present invention.

[FIG. 10] A block diagram showing a key configuration of a reactivity prediction system according to a third embodiment of the present invention.

[FIG. 11] An explanatory view showing an example of reaction condition presentation processing performed by a reaction condition presentation unit according to the third embodiment of the present invention.

[FIG. 12] A block diagram showing a key configuration of a reactivity prediction system according to a fourth embodiment of the present invention.

[FIG. 13] An explanatory view showing an example of reaction condition presentation processing performed by a reaction condition presentation unit according to the fourth embodiment of the present invention.

[FIG. 14] A block diagram showing an example of a functional configuration pertaining to $\Delta H$ prediction of a reactivity prediction system 40 according to a fifth embodiment of the present invention.

[FIG. 15] A flowchart showing an example of $\Delta H$ prediction processing performed by the reactivity prediction system 40 according to the fifth embodiment of the present invention.

[FIG. 16] A block diagram showing a key configuration of a reactivity prediction system 40 according to a sixth embodiment of the present invention.

[FIG. 17] An explanatory view showing an example of reaction condition presentation processing performed by a reaction condition presentation unit 67 according to the sixth embodiment of the present invention.

DESCRIPTION OF EMBODIMENTS

Hereinafter, a reactivity prediction system and reactivity prediction method according to embodiments of the present invention will be described in detail with reference to the drawings. In the following, the overview of the embodiments of the present invention will be described first, followed by a detailed description of each embodiment of the present invention.

[Overview]

In production of pharmaceuticals or fine chemical materials using organic chemical reactions, optimization of reaction conditions is important to improve production efficiency. The reaction conditions include, for example, selection of solvent types and reagent, a concentration, a temperature, and a reaction time. In the optimization process, conditions under which a high yield can be obtained are searched for to improve efficiency. In the optimization process, each item is optimized sequentially, and a yield and reaction rate measured by sampling the concentration of a product at a reaction time are mainly used as indicators thereof. For synthesis of a product, for example, a flow reactor which injects a first solution and a second solution into a supply device and mixes and synthesizes them with a mixer is used.

However, in the technique in the related art, a step of sampling a reaction fluid at a certain reaction time for determining the reaction rate from an experiment to, for example, perform extraction or measurement of the concentration of a product on the sampled reaction fluid is required, and it takes at least several hours of work to calculate the concentration under one condition. In the technique in the related art, it is necessary to repeat a trial and error process in which changing conditions and extracting are performed several tens of times or more depending on the number of optimization items. In addition, in the technique in the related art, more man-hours are required because concentration data under a plurality of temperature conditions are required.

In the above-described trial and error, it may take a long time to synthesize a target compound, the cost may increase, or in the worst case, it may not be possible to synthesize it. Therefore, the methods disclosed in Patent Documents 1 and 2 derive an optimal synthetic route by a quantum chemical calculation. In these methods, activation free energy ($\Delta G^{\ddagger}$) used as input data for predicting synthesis routes is obtained through a calculation. However, since this $\Delta G^{\ddagger}$ is not a measured value but a value calculated from a simulation, there is a large deviation from the measured value, which poses a problem.

In the embodiments of the present invention, activation free energy is predicted from input reaction conditions using a storage unit, which stores a learned model that has learned a relationship between measured activation free energy and reaction conditions under which the activation free energy is measured, and the learned model. Accordingly, activation free energy can be predicted with higher accuracy than that in the

related art.

[First embodiment]

First, a soft sensor (measurement device) that measures activation free energy will be described.

<Soft sensor>

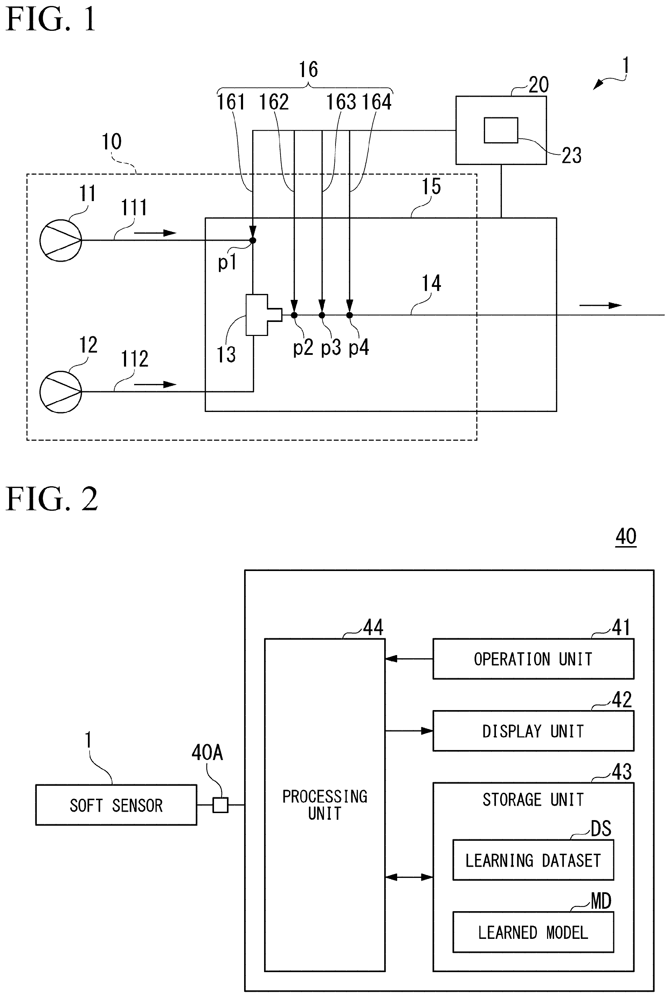

FIG. 1 is a block diagram showing a configuration example of a soft sensor 1 according to a first embodiment of the present invention. As shown in FIG. 1, the soft sensor 1 includes a first pump 11, a liquid feed tube 111, a second pump 12, a liquid feed tube 112, a mixer 13, a reaction tube 14, a temperature regulator 15, a temperature measurement unit 16, and a reaction analysis device 20. The temperature measurement unit 16 includes a first temperature measurement unit 161, a second temperature measurement unit 162, a third temperature measurement unit 163, and a fourth temperature measurement unit 164. A flow reactor 10 includes the first pump 11, the liquid feed tube 111, the second pump 12, the liquid feed tube 112, the mixer 13, and the reaction tube 14. The reaction analysis device 20 includes a CPU 23.

The flow reactor 10 shown in FIG. 1 includes: a plurality of supply flow paths (the first pump 11, the liquid feed tube 111, the second pump 12, and the liquid feed tube 112) that respectively supply a plurality of reactants to be used for chemical reactions; and the mixer 13 that is connected to those plurality of supply flow paths and mixes the plurality of reactants with each other, and is formed in the form of a flow path.

The first pump 11 is connected to a first introduction port of the mixer 13 through the liquid feed tube 111. The second pump 12 is connected to a second introduction port of the mixer 13 through the liquid feed tube 112. The mixer 13 includes two introduction ports and one discharge port. The discharge port of the mixer 13 is connected to the reaction tube 14 in which a reaction fluid obtained by mixing the plurality of reactants with each other circulates.

The temperature measurement unit 16 includes the first temperature measurement unit 161, the second temperature measurement unit 162, the third temperature measurement unit 163, and the fourth temperature measurement unit 164 arranged at a plurality of positions along the flow path before and after the mixer 13, for example, so that the temperature of the reaction fluid at the plurality of positions along the reaction tube 14 can be measured. The number of temperature measurement units 16 is not limited to four, and may be, for example, four or more.

The first temperature measurement unit 161 can be installed on an input side of the mixer 13, and the initial temperature (temperature of the reaction fluid at the discharge port of the mixer 13) of the reaction fluid obtained by mixing the plurality of the reactants with each other can be measured using the first temperature measurement unit 161.

The second temperature measurement unit 162, the third temperature measurement unit 163, and the fourth temperature measurement unit 164 are installed in the reaction tube 14 on the output side of the mixer 13. The temperature (temperature distribution) of the reaction fluid immediately after mixing (immediately after the start of the reaction) along the flow direction of the reaction fluid can be measured by these second temperature measurement unit 162 to fourth temperature measurement unit 164. The expression "immediately after the start of the reaction" does not mean immediately after the actual start of the reaction of the reaction fluid, but means immediately after the placement of the reaction fluid in a state where the reaction has started (immediately after the plurality of the reactants are mixed with each other in the first embodiment).

The liquid feed tube 111, the liquid feed tube 112, the mixer 13, and the reaction tube 14 are installed in the temperature regulator 15.

<Operation of soft sensor>

The soft sensor 1 identifies a reaction state of a reaction fluid in a flow synthesis type chemical reaction device. The soft sensor 1 measures a temperature before a reaction and temperatures at a plurality of sites after a reaction, and reaction parameters including activation free energy ($\Delta G^{\ddagger}$) are calculated based on the measured temperatures. Reactants placed in the first pump 11 and the second pump 12 may be liquids or gases. The reaction parameters indicate a reaction state of a reaction fluid, and are, for example, parameters that affect the temperature distribution of a chemical reaction field as will be described below. Products produced by the soft sensor 1 are, for example, peptide synthesis products.

A first reactant is placed in the first pump 11. The first pump 11 supplies the placed first reactant to the mixer 13 through the liquid feed tube 111 at a first flow velocity and a first flow rate, for example.

A second reactant is placed in the second pump 12. The second pump 12 supplies the placed second reactant to the mixer 13 through the liquid feed tube 112 at a second velocity and a second flow rate, for example.

The mixer 13 mixes the first reactant supplied from the first pump 11 with the second reactant supplied from the second pump 12 and supplies the mixed product to the reaction tube 14.

The product is supplied from the discharge port of the mixer 13 to the reaction tube 14. In the space inside the mixer 13, mixing of the first reactant with the second reactant is started. Then, in the flow reactor 10, a reaction occurs from the inside of the mixer 13 to the inside of the reaction tube 14, and a solution including, for example, the product moves through the reaction tube 14. Then, in the flow reactor 10, the solution including, for example, the product is discharged to the outside of the reaction tube 14 through the reaction tube 14.

The temperature regulator 15 is, for example, a constant-temperature water tank and regulates the temperature of the mixer 13 and the reaction tube 14 to a predetermined temperature according to a control of the reaction analysis device 20.

The temperature measurement unit 16 is a sensor for measuring the temperature of a chemical reaction field, and is, for example, a thermocouple. The temperature measurement unit 16 may be, for example, an optical temperature sensor which is a non-contact type. The temperature measurement unit 16 measures the temperature distribution of a reaction fluid along the reaction tube 14 and outputs the temperature information indicating the measured temperature (actually measured temperature distribution) to the reaction analysis device 20. The chemical reaction field is an area where a chemical reaction of reactants mixed downstream of the mixer 13 occurs.

The first temperature measurement unit 161 is installed at a position p1 before a reaction. The installation site may be at least one of the first pump 11 side and the second pump 12 side, or may be both of the first pump 11 side and the second pump 12 side. When the first temperature measurement unit 161 is installed on both the first pump 11 side and the second pump 12 side, an average value of the temperature on the first pump 11 side and the temperature on the second pump 12 side may be output to the reaction analysis device 20. The average value may be calculated by the reaction analysis device 20. It is not necessarily essential to measure the temperature of the upstream side of the mixer 13. For example, when the temperature of a reactant is kept constant, the first temperature measurement unit 161 is unnecessary.

The second temperature measurement unit 162 is installed at a position p2 after a reaction. The position p2 is a position closest to the discharge port of the mixer 13 among the positions p2 to p4. The second temperature measurement unit 162 measures the temperature of the position p2 and outputs the information indicating the measured temperature to the reaction analysis device 20.

The third temperature measurement unit 163 is installed at a position p3 after a reaction. The position p3 is a position between the position p2 and a position p4, and the distance from the discharge port of the mixer 13 is longer than that of the position p2. The third temperature measurement unit 163 measures the temperature of the position p3 and outputs the information indicating the measured temperature to the reaction analysis device 20.

The fourth temperature measurement unit 164 is installed at the position p4 after a reaction. The position p4 is a position farthest from the discharge port of the mixer 13 among the positions p2 to p4. The fourth temperature measurement unit 164 measures the temperature of the position p4 and outputs the information indicating the measured temperature to the reaction analysis device 20.

The reaction analysis device 20 controls the temperature regulator 15. The reaction analysis device 20 controls the flow rate of the first pump 11 and the second pump 12. The reaction analysis device 20 acquires the information indicating the measured temperature output by the temperature measurement unit 16. The reaction analysis device 20 identifies a reaction state of a reaction fluid obtained by mixing a plurality of reactants with each other using the information indicating the acquired temperature. The reaction state refers to, for example, the reaction rate of a reaction fluid, the concentration of a plurality of reactants, and the concentration or yield of a product contained in the reaction fluid. The reaction analysis device 20 analyzes a reaction by estimating, for example, a function of a position and a temperature. The estimation method or the like will be described below.

The configuration shown in FIG. 1 is an example and is not restrictive. For example, a first product may be produced by mixing a first reactant with a second reactant in a first mixer, and a second product may be produced by mixing the first product with a third reactant in a second mixer. In this case, by mounting temperature measurement unit(s) before and after (upstream and downstream) of the first mixer of only downstream thereof and mounting temperature measurement unit(s) before and after (upstream and downstream) of the second mixer or only downstream thereof, the temperature distribution of a reaction fluid immediately after mixing the first reactant with the second reactant using the first mixer may be detected and the temperature distribution of a reaction fluid immediately after mixing the first product with the third reactant using the second mixer may be detected.

<Method for calculating reaction parameters from temperature distribution of reaction field>

Next, a method for calculating reaction parameters from temperature distribution of a reaction field will be described.

A function $T_n(x)$ is a solution of a governing equation estimating temperature distribution of a reaction fluid immediately after mixing, and is a function of a temperature with respect to a position x. For example, $T_n(p1)$ is an estimation value of a temperature at the position p1 according to the function $T_n(x)$. $T_n(p2)$ is an estimation value of a temperature at the position p2 according to the function $T_n(x)$. $T_n(p3)$ is an estimation value of a temperature at the position p3 according to the function $T_n(x)$. $T_n(p4)$ is an estimation value of a temperature at the position p4 according to the function $T_n(x)$.

The reaction analysis device 20 compares actually measured temperature distribution obtained by actually measuring the temperature of a reaction fluid using the temperature measurement unit 16 with estimated temperature distribution obtained by estimating temperature distribution of the reaction fluid immediately after mixing, and sets reaction parameters for which the difference between the estimated value and the actual measurement value is within a predetermined value.

Here, the reaction parameters and the function $T_n(x)$ will be described.

The function $T_n(x)$ is a solution of a governing equation estimating temperature distribution of a reaction fluid immediately after mixing, and is a function of the position x and reaction parameters $\Delta H$ and $\Delta G^{\ddagger}$ as shown in Equation (1) below.

[Equation 1]

$$T_n(x) \approx f\left(x, \Delta H, \Delta G^{\ddagger}\right) \qquad \cdots (1)$$

The reaction parameter $\Delta H$ is a reaction molar enthalpy (kJ/mol). The reaction molar enthalpy is a quantity representing reaction heat per mole (per unit amount of substance). The reaction parameter $\Delta H$ relates to a peak value of temperature distribution of a reaction fluid immediately after mixing.

The reaction parameter $\Delta G^{\ddagger}$ is activation free energy (kJ/mol). The activation free energy is a difference in free energy between before a reaction and a reaction transition state, and indicates the temperature dependence of the reaction rate. The reaction parameter $\Delta G^{\ddagger}$ relates to a peak value and peak position of temperature distribution of a reaction fluid immediately after mixing.

Next, processing procedure example of reaction analysis in the soft sensor 1 will be described.

The reaction analysis device 20 acquires information on the temperature measured by the temperature measurement unit 16 (actually measured temperature distribution obtained by actually measuring the temperature of a reaction fluid). Accordingly, the reaction analysis device 20 reads a temperature distribution measurement value $T_0(x)$. The reaction analysis device 20 stores the position x at which the temperature measurement unit 16 is installed.

Next, the reaction analysis device 20 temporarily sets reaction parameters $\Delta H_n$ and $\Delta G^{\ddagger}_n$ and calculates a temperature distribution calculation value $T_n(x)$ (acquires estimated temperature distribution obtained by estimating temperature distribution of a reaction fluid immediately after mixing). When the processing is at the first time, the reaction analysis device 20 calculates a temperature distribution calculation value $T_1(x)$ by setting reaction parameters $\Delta H_1$ and $\Delta G^{\ddagger}_1$ to initial values stored by the unit itself.

Next, the reaction analysis device 20 calculates a difference $\Delta tm$ between an estimated temperature value at each position pm (m is, for example, an integer of 1 to 4) estimated by the function $T_n(x)$ and an actually measured temperature value Tm at each position pm actually measured by the temperature measurement unit 16 (compares the actually measured temperature distribution with the estimated temperature distribution). When there are 4 temperature measurement sites, the reaction analysis device 20 determines whether or not $\Delta t1$ (=T1-$T_n$(p1)), $\Delta t2$ (=T2-$T_n$(p2)), $\Delta t3$ (=T3-$T_n$(p3)), $\Delta t4$ (=T4-$T_n$(p4)) are all within a predetermined value stored by the device itself to determine whether or not $T_n(x)$ is almost consistent with $T_0(x)$.

When the reaction analysis device 20 determines that $T_n(x)$ does not almost correspond to $T_0(x)$, it adjusts the reaction parameter values. For example, when $\Delta t1$ to $\Delta t4$ are all positive, the reaction parameter $\Delta H$ is set to a value greater than $\Delta H_1$ so that the height of the maximum value is increased. For example, when $\Delta t1$ to $\Delta t4$ are all negative, the reaction parameter $\Delta H$ is set to a value smaller than $\Delta H_1$ so that the height of the maximum value is reduced. In addition, if only $\Delta t2$ is negative and the others are positive (for example, if the peak position of estimated temperature distribution is located backward compared to measured temperature distribution), $\Delta G^{\ddagger}_1$ is set to be small so that the peak position of the estimated temperature distribution is forward.

When the reaction analysis device 20 determines that $T_n(x)$ almost corresponds to $T_0(x)$ or that $T_n(x)$ almost corresponds to $T_0(x)$ by adjusting the above-described reaction parameters, it outputs the reaction parameters $\Delta H$ and $\Delta G^{\ddagger}$.

In addition, the reaction analysis device 20 calculates a concentration distribution P(x) of a product and a reactant and outputs the calculated concentration distribution P(x) of the product and the reactant.

The concentration distribution P(x) of the product and the reactant calculated and output by the reaction analysis device 20 is obtained by solving a differential equation of Equation (2) below when the reaction parameters are $\Delta H$ and $\Delta G^{\ddagger}$.

[Equation 2]

$$\frac{d[P]}{dt} = \frac{k_B T}{h}[A][B]\exp\left(-\frac{\Delta G^{\ddagger}}{RT}\right) \qquad \cdots (2)$$

In Equation (2), [A] is a concentration of a first reactant, [B] is a concentration of a second reactant, h is the Planck constant ($6.62607004 \times 10^{-34}$ ($m^2$ kg/s)), $k_B$ is the Boltzmann constant ($1.380649 \times 10^{-23}$ ($JK^{-1}$)), R is a gas constant, and T is a temperature of a fluid.

In this manner, in the soft sensor 1, the reaction parameters including the activation free energy ($\Delta G^{\ddagger}$) can be calculated using the actual measurement values.

In addition, by estimating the concentration, the yield of a product at any position and at any time in the flow reactor 10 can be calculated.

<Reactivity prediction system>

FIG. 2 is a block diagram showing a key configuration of a reactivity prediction system 40 according to the first embodiment of the present invention. As shown in FIG. 2, the reactivity prediction system 40 of the present embodiment includes an operation unit 41, a display unit 42, a storage unit 43, and a processing unit 44. Such a reactivity prediction system 40 is realized by, for example, a desktop type, notebook type, or tablet type computer, or a workstation. In addition, the reactivity prediction system 40 includes a first interface 40A to which the above-described soft sensor 1 can be connected. The first interface 40A is realized by, for example, a connector, a socket, or a coupling.

For example, the operation unit 41 includes an input device such as a keyboard or a pointing device, and outputs instructions (instructions for the reactivity prediction system 40) in response to an operation of a worker using the reactivity prediction system 40 to the processing unit 44. For example, the display unit 42 includes a display device such as a liquid crystal display device, and displays various information output from the processing unit 44. The operation unit 41 and the display unit 42 may be physically separated of physically integrated like a touch panel type liquid crystal display device having both a display function and an operation function.

For example, the storage unit 43 includes a storage device such as an HDD (hard disk drive) or an SSD (solid state drive) and stores various data for use in the reactivity prediction system 40. The storage unit 43 stores, for example, a learning dataset DS and a learned model MD. The storage unit 43 may store a program that realizes the function of the reactivity prediction system 40.

The learning dataset DS is data obtained from the soft sensor 1. This learning dataset DS is, for example, data including activation free energy ($\Delta G^{\ddagger}$) measured by the soft sensor 1 and reaction conditions (first datasets) under which the activation free energy is measured. The learned model MD is a model that has learned a relationship between the measured activation free energy ($\Delta G^{\ddagger}$) and the reaction conditions under which the activation free energy is measured, and is generated by performing machine learning using the learning dataset DS. The details of the learned model MD will be described below.

For example, the processing unit 44 is realized by a CPU (central processing unit), RAM (random-access memory), or the like, and collectively controls the operation of the reactivity prediction system 40 based on the operation instructions input from the operation unit 41. For example, the processing unit 44 performs processing to generate a learned model MD from a learning dataset DS, processing to predict activation free energy from reaction conditions input from the operation unit 41 using the learned model MD, and other processing steps. The processing unit 44 can also perform processing to display predicted activation free energy and other outputs on the display unit 42 based on operation instructions input from the operation unit 41.

The function of the reactivity prediction system 40 may be realized by executing a program (including a program recorded on a recording medium not shown in the drawing) that realizes the function using hardware such as CPUs. That is, the function of the reactivity prediction system 40 may be realized by cooperation between software and hardware resources. As a matter of course, the function of the reactivity prediction system 40 may be realized using hardware such as FPGA, LSI, and ASIC.

<Generation of learned model>

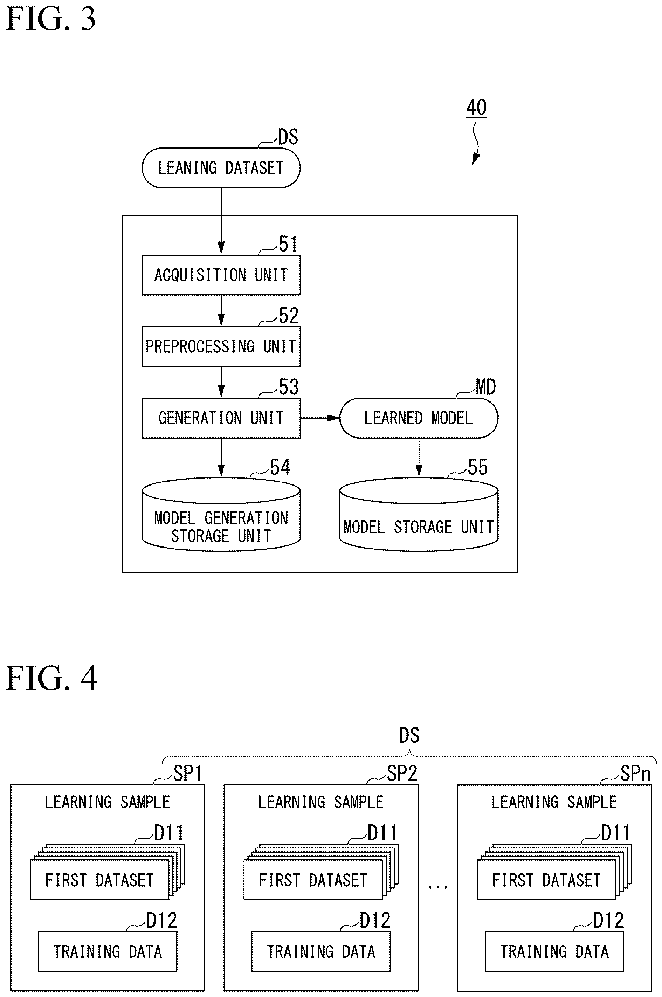

FIG. 3 is a block diagram showing an example of a functional configuration pertaining to generation of a learned model MD of the reactivity prediction system 40 according to the first embodiment of the present invention. As shown in FIG. 3, the reactivity prediction system 40 includes an acquisition unit 51, a preprocessing unit 52, a generation unit 53, a model generation storage unit 54, and a model storage unit 55. The acquisition unit 51, the preprocessing unit 52, and the generation unit 53 are realized by the processing unit 44 shown in FIG. 2. In other words, the processing unit 44 executes a model generation program stored in the storage unit 43 to realize the functions of the acquisition unit 51, the preprocessing unit 52, and the generation unit 53. The model generation storage unit 54 and the model storage unit 55 are realized by the storage unit 43 shown in FIG. 2.

The acquisition unit 51 acquires a learning dataset DS. Examples of modes in which the acquisition unit 51 acquires a learning dataset DS include a mode of acquiring training data (measured activation free energy) input from the soft sensor 1 and reaction conditions input from the operation unit 41 (reaction conditions under which activation free energy is measured), for example. The training data input from the soft sensor 1 and the reaction conditions input from the operation unit 41 may be temporarily stored in the storage unit 43 as learning dataset DS, and the acquisition unit 51 may acquire the learning dataset DS temporally stored in the storage unit 43.

FIG. 4 is an explanatory view showing an example of learning datasets DS used in the first embodiment of the present invention. As shown in FIG. 4, the learning datasets DS used in the present embodiment include learning samples SP1 to SPn (n is an integer of 2 or more). The learning samples SP1 to SPn are obtained from past measurement results and include first datasets D11 and training data D12.

The labeled training data D12 is activation free energy ($\Delta G^{\ddagger}$) measured by the soft sensor 1. The first datasets D11 are reaction conditions under which the activation free energy is measured, and is data obtained by inputting the reaction conditions to the reactivity prediction system 40 by operating the operation unit 41, for example. The reaction conditions of the first datasets D11 may include at least one of a reactant, a solvent, a catalyst, an additive, a reagent, a temperature, and a reaction time. The learning samples SP1 to SPn include the first datasets D11 and the training data D12.

Returning to FIG. 3, the preprocessing unit 52 performs preprocessing necessary to create a learned model MD for the learning datasets DS acquired by the acquisition unit 51. If the preprocessing for the learning datasets DS is not required, the preprocessing unit 52 is not be repeated.

The generation unit 53 generates a learned model MD by training a learning model using the learning datasets DS preprocessed by the preprocessing unit 52. The learned model MD generated by the generation unit 53 is a model represented using a neural network, for example. However, the learned model MD is not limited to the model represented using a neural network, and can be a model represented using other algorithms such as a random forest and a support vector machine, for example.

The model generation storage unit 54 stores learning models and parameters that are being trained by the generation unit 53. The model storage unit 55 stores the learned model MD generated by the generation unit 53.

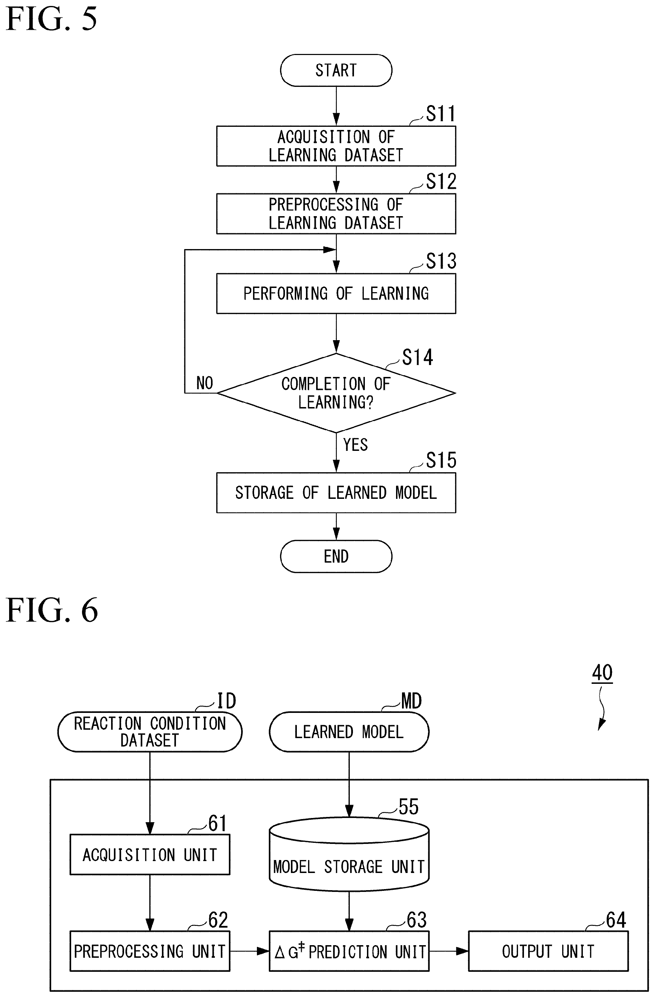

FIG. 5 is a flowchart showing an example of model generation processing performed by the reactivity prediction system 40 according to the first embodiment of the present invention. The processing of the flowchart show in FIG. 5 is started such that, for example, an operator using the reactivity prediction system 40 operates the operation unit 41 of the reactivity prediction system 40 to give an instruction to start learning. For the sake of simplicity of explanation, the model generation storage unit 54 shown in FIG. 3 is assumed to store learning models and parameters.

When the processing of the flowchart shown in FIG. 5 begins, first, the acquisition unit 51 of the reactivity prediction system 40 acquires the learning datasets DS (Step S11). Next, the preprocessing unit 52 of the reactivity prediction system 40 performs preprocessing of the acquired learning datasets DS (Step S12).

Subsequently, the generation unit 53 of the reactivity prediction system 40 performs machine learning using the preprocessed learning datasets DS (Step S13). Here, since the learning datasets DS include the training data D12, the generation unit 53 performs supervised learning. Subsequently, the generation unit 53 determines whether or not the machine learning is completed (Step S14).

When it is determined that the machine learning is not completed (when the determination result of Step S14 is "NO"), the generation unit 53 continues the machine learning (Step S13). For example, among the learning samples SP1 to SPn included in the learning dataset DS, one which has not been used for machine learning is used for machine learning. Meanwhile, when it is determined that the machine learning is completed (when the determination result of Step S14 is "YES"), the generation unit 53 stores the produced learned model MD in the model storage unit 55 (Step S15). The series of processing steps shown in FIG. 5 is completed with the above-described processing.

In the present embodiment, an example in which the learned model MD is generated in the reactivity prediction system 40 is described. However, the learned model MD may be generated in other devices different from the reactivity prediction system 40. When the learned model MD is generated in another device, the learned model MD may be supplied from the other device and stored in the model storage unit 55.

<$\Delta G^{\ddagger}$ Prediction>

FIG. 6 is a block diagram showing an example of a functional configuration pertaining to $\Delta G^{\ddagger}$ prediction of the reactivity prediction system 40 according to the first embodiment of the present invention. As shown in FIG. 6, the reactivity prediction system 40 includes an acquisition unit 61, a preprocessing unit 62, a $\Delta G^{\ddagger}$ prediction unit 63, and an output unit 64, in addition to the model storage unit 55. The acquisition unit 61 to the $\Delta G^{\ddagger}$ prediction unit 63 are realized by the processing unit 44 shown in FIG. 2. In other words, the processing unit 44 executes a program stored in the storage unit 43 to realize the functions of the acquisition unit 61 to $\Delta G^\ddagger$ prediction unit 63. The model storage unit 55 is realized by the storage unit 43 shown in FIG. 2, and the output unit 64 is realized by the display unit 42 shown in FIG. 2.

The acquisition unit 61 operates the operation unit 41 of the reactivity prediction system 40 shown in FIG. 2 to acquire input reaction conditions (reaction condition datasets ID), for example. When the reaction condition datasets ID are stored in the storage unit 43, the acquisition unit 61 may acquire the reaction condition datasets ID from the storage unit 43. The reaction condition datasets ID may include at least a reactant, a catalyst, and a solvent.

The preprocessing unit 62 performs preprocessing necessary for predicting $\Delta G^\ddagger$ on the reaction condition datasets ID acquired by the acquisition unit 61. If the preprocessing for the reaction condition datasets ID is not required, the preprocessing unit 62 is not be repeated.

The $\Delta G^\ddagger$ prediction unit 63 performs $\Delta G^\ddagger$ prediction using the learned model MD stored in the model storage unit 55 and the reaction condition datasets ID preprocessed by the preprocessing unit 62. The output unit 64 displays prediction results of the $\Delta G^\ddagger$ prediction unit 63.

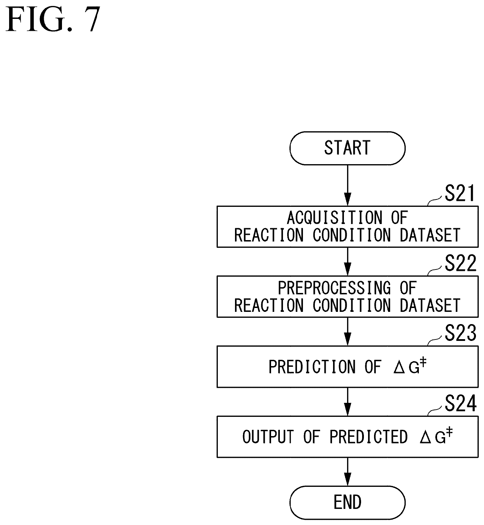

FIG. 7 is a flowchart showing an example of $\Delta G^\ddagger$ prediction processing performed by the reactivity prediction system 40 according to the first embodiment of the present invention. The processing of the flowchart shown in FIG. 7 is started such that, for example, an operator using the reactivity prediction system 40 operates the operation unit 41 of the reactivity prediction system 40 to give an instruction to start $\Delta G^\ddagger$ prediction processing.

When the processing of the flowchart shown in FIG. 7 begins, first, the acquisition unit 61 of the reactivity prediction system 40 acquires the reaction condition datasets ID to be predicted (Step S21). For example, the acquisition unit 61 acquires the reaction condition datasets ID input from the operation unit 41.

Next, the preprocessing unit 62 of the reactivity prediction system 40 performs preprocessing of the acquired reaction condition datasets ID (Step S22).

Next, the $\Delta G^\ddagger$ prediction unit 63 of the reactivity prediction system 40 performs $\Delta G^\ddagger$ prediction using the learned model MD stored in the model storage unit 55 and the reaction condition datasets ID preprocessed by the preprocessing unit 62 (Step S23).

Subsequently, the output unit 64 of the reactivity prediction system 40 outputs the predicted $\Delta G^\ddagger$ (Step S24). Accordingly, the predicted $\Delta G^\ddagger$ in the $\Delta G^\ddagger$ prediction unit 63 is displayed in the output unit 64.

As described above, the reactivity prediction system 40 according to the present embodiment includes: the storage unit 43 that stores a learned model MD that has learned a relationship between measured activation free energy ($\Delta G^\ddagger$) and reaction conditions under which the activation free energy is measured; and the processing unit 44 that predicts activation free energy from input reaction conditions (reaction condition datasets ID) using the learned model MD. According to this configuration, since $\Delta G^\ddagger$ is predicted using machine learning from $\Delta G^\ddagger$ actually measured by the soft sensor 1, a more reliable $\Delta G^\ddagger$ than $\Delta G^\ddagger$ derived only by a quantum chemical calculation is obtained.

In addition, the reactivity prediction system 40 according to the present embodiment further includes: the generation unit 53 that generates the learned model MD using the measured activation free energy as training data D12 and the reaction conditions under which the activation free energy is measured as a first dataset D11 in the reactivity prediction system 40 according to the first aspect of the present invention. According to this configuration, the learned model MD can be generated inside the reactivity prediction system 40. The learned model MD may be generated outside the reactivity prediction system 40.

In addition, in the reactivity prediction system 40 according to the present embodiment, the reaction conditions of the first dataset D11 include at least one of a reactant, a solvent, a catalyst, an additive, a reagent, a temperature, and a reaction time. According to this configuration, a more reliable $\Delta G^\ddagger$ can be predicted.

In addition, the reactivity prediction system 40 according to the present embodiment further includes: the first interface 40A connectable to the soft sensor 1 (measurement device) that measures the activation free energy. According to this configuration, the measured activation free energy ($\Delta G^\ddagger$) can be directly acquired from the soft sensor 1 through the first interface 40A.

In addition, a reactivity prediction method according to the present embodiment includes: a first step (Step S21) of inputting reaction conditions; and a second step (Step S23) of predicting activation free energy from the reaction conditions input in the first step using a learned model MD that has learned a relationship between measured activation free energy and reaction conditions under which the activation free energy is measured. According to this configuration, since $\Delta G^\ddagger$ is predicted using machine learning from $\Delta G^\ddagger$ actually measured by the soft sensor 1, a more reliable $\Delta G^\ddagger$ than $\Delta G^\ddagger$ derived by only a quantum chemical calculation is obtained.

In this manner, according to the present embodiment described above, activation free energy can be predicted with higher accuracy than that in the related art.

[Second embodiment]

<Reactivity prediction system>

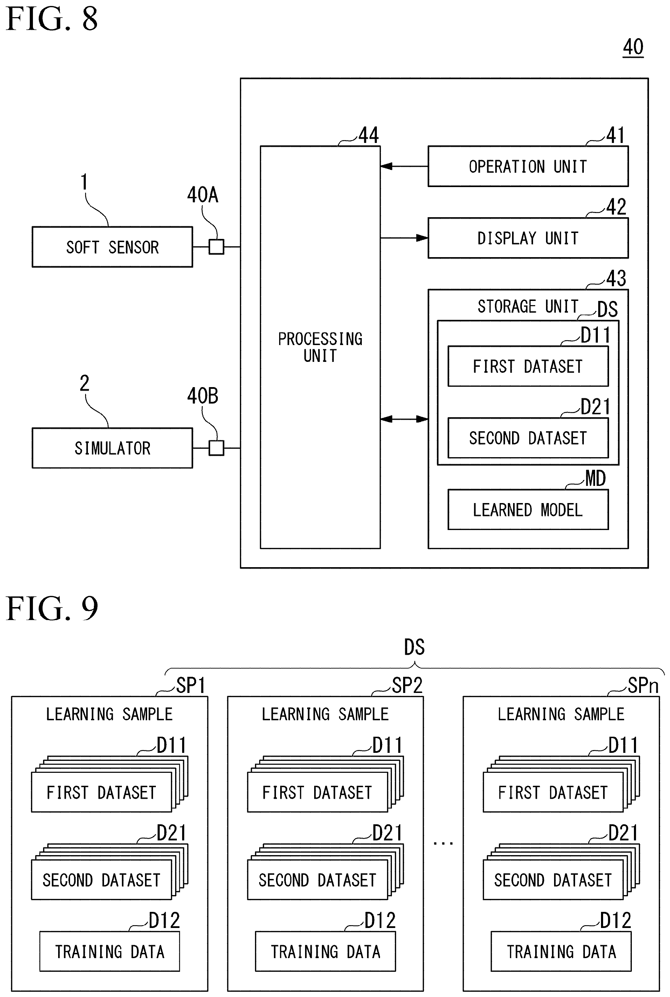

FIG. 8 is a block diagram showing a key configuration of a reactivity prediction system 40 according to a second embodiment of the present invention. **In** FIG. 8, the same configuration as that shown in FIG. 2 will be denoted by the same reference numerals. As shown in FIG. 8, the reactivity prediction system 40 according to the second embodiment further includes: a second interface 40B to which a simulator 2 (calculation device) can be connected. The second interface 40B is realized by, for example, a connector, a socket, or a coupling.

The simulator 2 performs a quantum chemical calculation to calculate a feature amount relating to a reaction. The simulator 2 calculates at least one of a dipole moment, atomic charge, and frequency as a feature amount. The simulator 2 may further calculate, for example, an energy gap between HOMO (highest occupied molecular orbital) and LUMO (lowest unoccupied molecular orbital) as a feature amount.

In addition to the above-described first dataset D11, a second dataset D21 is stored in a storage unit 43 as a learning dataset DS. The second dataset D21 is data obtained from the simulator 2. The second dataset D21 includes above-described feature amount and calculation conditions thereof. The feature amount included in the second dataset D21 may be a feature amount that cannot be obtained by an experiment with the above-described soft sensor 1. Accordingly, $\Delta G^{\ddagger}$ can be efficiently obtained without performing experiments for all reaction conditions.

FIG. 9 is an explanatory view showing an example of learning datasets used in the second embodiment of the present invention. As shown in FIG. 9, the learning dataset DS used in the present embodiment includes learning samples SP1 to SPn in the same manner as the learning dataset DS shown in FIG. 4. However, each of the learning samples SP1 to SPn includes second datasets D21 in addition to the first datasets D11 and the training data D12.

The reactivity prediction system 40 generates a learned model MD using the learning dataset DS shown in FIG. 9. In other words, the learned model MD is trained to learn a relationship (second dataset D21) between a feature amount relating to a reaction obtained by a quantum chemical calculation and calculation conditions under which the quantum chemical calculation is performed in addition to the relationship (first dataset D11) between measured activation free energy (training data D12) and reaction conditions under which the activation free energy is measured.

In addition, the reactivity prediction system 40 predicts activation free energy from the learned model MD generated using the learning dataset DS shown in FIG. 9 and input reaction conditions (reaction condition datasets ID). The functional configuration and the model generation processing relating to the generation of the learned model MD of the reactivity prediction system 40 and the functional configuration and the state determination processing relating to the prediction of activation free energy are the same as those of the first embodiment, and therefore the description thereof will not be repeated.

As described above, in the reactivity prediction system 40 according to the second embodiment, the learned model MD is trained to learn a relationship between a feature amount relating to a reaction obtained by a quantum chemical calculation and calculation conditions under which the quantum chemical calculation is performed in addition to the relationship between measured activation free energy and reaction conditions under which the activation free energy is measured. According to this configuration, by predicting $\Delta G^{\ddagger}$ using machine learning from $\Delta G^{\ddagger}$ obtained for some reaction conditions by the soft sensor 1 and a feature amount calculated by a quantum chemical calculation, $\Delta G^{\ddagger}$ can be efficiently obtained without performing experiments for all reaction conditions. In addition, $\Delta G^{\ddagger}$ calculated by a quantum chemical calculation includes an error, in the second embodiment, a more reliable $\Delta G^{\ddagger}$ than that in the related art is obtained using $\Delta G^{\ddagger}$ actually measured from the soft sensor 1 for machine learning.

In addition, the reactivity prediction system 40 according to the second embodiment further includes: a generation unit 53 that generates the learned model MD using the measured activation free energy as training data D12, the reaction conditions under which the activation free energy is measured as a first dataset D11, and the feature amount and the calculation conditions as a second dataset D21. According to this configuration, the learned model MD can be generated inside the reactivity prediction system 40. The learned model MD may be generated outside the reactivity prediction system 40.

In addition, in the reactivity prediction system 40 according to the second embodiment, the feature amount of the second dataset D21 includes at least one of a dipole moment, atomic charge, frequency, and a molecular orbital. According to this configuration, a more reliable $\Delta G^{\ddagger}$ can be predicted.

In addition, the reactivity prediction system 40 according to the second embodiment further includes: a second interface 40B connectable to a simulator 2 (calculation device) that performs the quantum chemical calculation. According to this configuration, it is possible to directly acquire the feature amount relating to a reaction from the simulator 2 through the second interface 40B.

[Third embodiment]

<Reactivity prediction system>

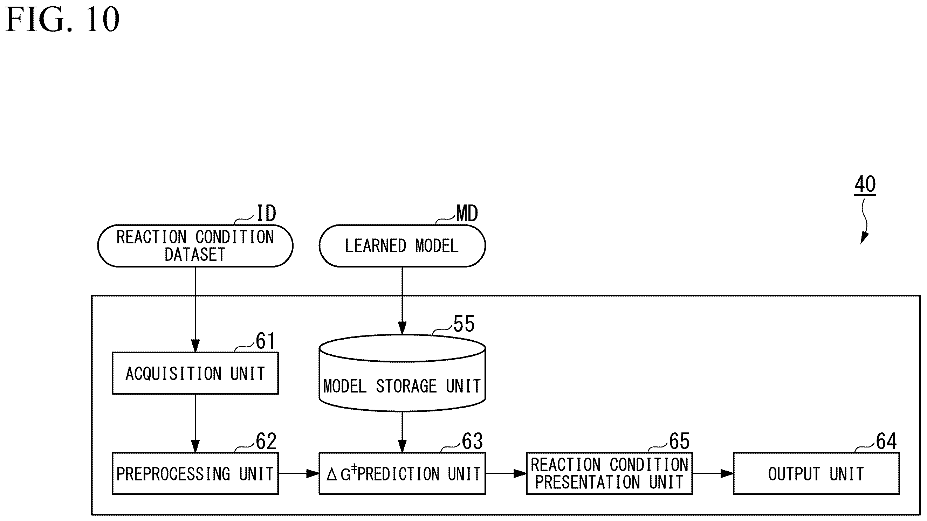

FIG. 10 is a block diagram showing a key configuration of a reactivity prediction system 40 according to a third embodiment of the present invention. In FIG. 10, the same configuration as that shown in FIG. 6 will be denoted by the same reference numerals. As shown in FIG. 10, the reactivity prediction system 40 according to the third embodiment further includes a reaction condition presentation unit 65 that presents predetermined reaction conditions based on activation free energy predicted by a $\Delta G^{\ddagger}$ prediction unit 63. The reaction condition presentation unit 65 is realized by the processing unit 44 shown in FIG. 2. In other words, the processing unit 44 executes a program stored in the storage unit 43 to realize the function of the reaction condition presentation unit 65.

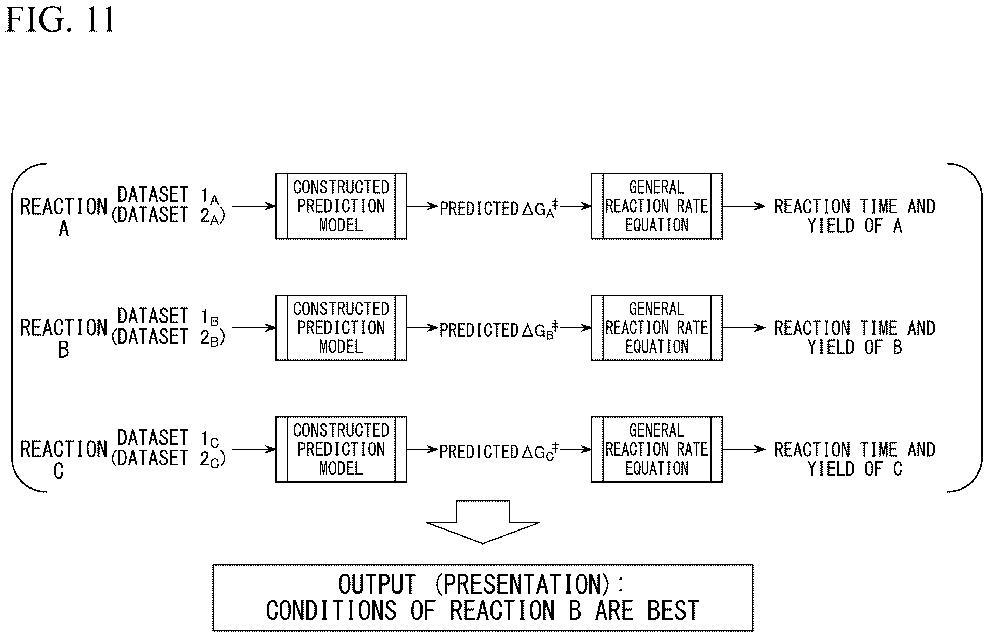

FIG. 11 is an explanatory view showing an example of reaction condition presentation processing performed by a reaction condition presentation unit 65 according to the third embodiment of the present invention. The reaction condition presentation processing shown in FIG. 11 presents reaction conditions expected to have the lowest activation free energy among the activation free energy predicted by the $\Delta G^{\ddagger}$ prediction unit 63. For example, the reaction condition presentation unit 65 compares $\Delta G^{\ddagger}$ among reactions A to C and executes an operation to select conditions (combination of reactants) with the lowest value.

Specifically, as shown in FIG. 10, the acquisition unit 61 acquires the reaction conditions (datasets $1_A$ to $1_C$ (datasets $2_A$ to $2_C$) shown in FIG. 11) input for each of the reactions A to C as the above-described reaction condition datasets ID. The datasets $1_A$ to $1_C$ may be reaction conditions including at least a reactant, a catalyst, and a solvent. When inputting the datasets $2_A$ to $2_C$, the datasets $2_A$ to $2_C$ may be feature amounts including at least one of a dipole moment, atomic charge, frequency, and a molecular orbital.

The preprocessing unit 62 performs preprocessing necessary for predicting $\Delta G^{\ddagger}$ on the reaction conditions acquired by the acquisition unit 61. If the preprocessing for the reaction condition datasets ID is not required, the preprocessing unit 62 is not be repeated. The $\Delta G^{\ddagger}$ prediction unit 63 performs $\Delta G^{\ddagger}$ prediction using the learned model MD stored in the model storage unit 55 and the reaction conditions preprocessed by the preprocessing unit 62.

The reaction condition presentation unit 65 presents reaction conditions expected to have the lowest activation free energy among the activation free energy predicted by the $\Delta G^{\ddagger}$ prediction unit 63. In the example of FIG. 11, the reaction condition presentation unit 65 presents the conditions of the reaction B having the lowest $\Delta G^{\ddagger}$. The reaction condition presentation unit 65 may present $\Delta G^{\ddagger}$ of the reactions A to C as a reference, or may present reaction times and yields of the reactions A to C from a general reaction rate equation using Equation (2) described above. An output unit 64 displays presentation results of the reaction condition presentation unit 65.

As described above, the reactivity prediction system 40 according to the third embodiment includes: the reaction condition presentation unit 65 that presents predetermined reaction conditions based on activation free energy predicted by the $\Delta G^{\ddagger}$ prediction unit 63 of the processing unit 44. The reaction condition presentation unit 65 presents, as the above-described predetermined reaction conditions, reaction conditions expected to have the lowest activation free energy among the activation free energy predicted by the $\Delta G^{\ddagger}$ prediction unit 63 of the processing unit 44. According to this configuration, by comparing $\Delta G^{\ddagger}$ and selecting the conditions having the lowest value (combination of reactants), it is possible to present reaction conditions having a short reaction time and a high yield.

[Fourth embodiment]

<Reactivity prediction system>

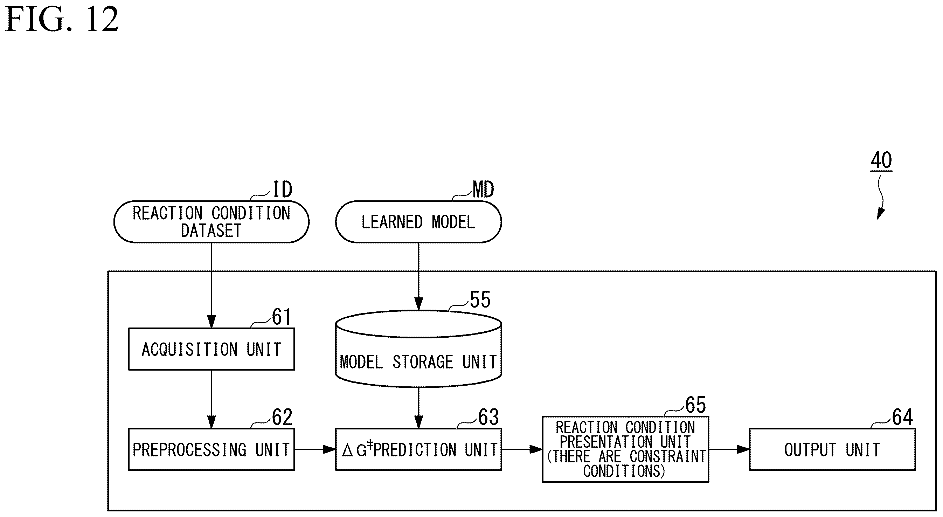

FIG. 12 is a block diagram showing a key configuration of a reactivity prediction system 40 according to a fourth embodiment of the present invention. In FIG. 12, the same configuration as that shown in FIG. 10 will be denoted by the same reference numerals. As shown in FIG. 12, the reaction condition presentation unit 65 according to the fourth embodiment presents predetermined reaction conditions based on the activation free energy predicted by the $\Delta G^{\ddagger}$ prediction unit 63 and predetermined constraint conditions.

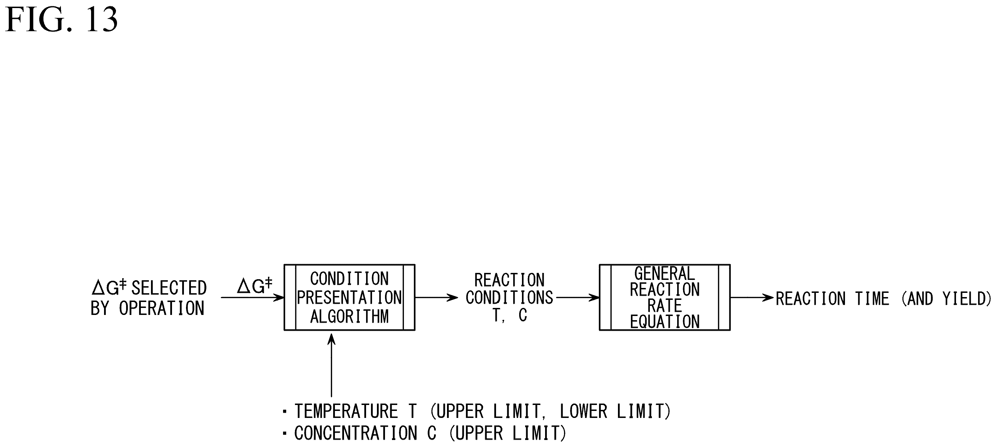

FIG. 13 is an explanatory view showing an example of reaction condition presentation processing performed by a reaction condition presentation unit 65 according to the fourth embodiment of the present invention. The reaction condition presentation processing shown in FIG. 13 presents reaction conditions (a reaction field temperature and a concentration of a reactant) that can be implemented within the constraint conditions and that allow a reaction to be completed within a reaction time determined by flow path restrictions, based on the selected $\Delta G^{\ddagger}$.

In other words, in the flow synthesis by the flow reactor 10 shown in FIG. 1, the interior of the flow paths becomes a reaction field (constraint). When the flow paths of the flow reactor 10 have a constant flow rate and inner diameter, the time for which a reaction can occur is determined by the length of the flow paths. However, the flow paths cannot be infinitely long. Accordingly, it is necessary to present conditions under which a reaction can be completed within a fixed reaction time. In the presentation of the reaction conditions here, a reaction time t, a reaction field temperature T, and a concentration C of a reactant are presented as the conditions in addition to the presentation in the third embodiment.

In other words, the constraint conditions may include at least one of a reaction time, a reaction field temperature, and a concentration of a reactant.

The reaction time t can be obtained by a function t (T, C) of the reaction field temperature T and the concentration C of a reactant. The upper limit value and the lower limit value of the reaction field temperature T are set in advance as constraints. The upper limit value of the concentration C of a reactant is set in advance. In other words, in the fourth embodiment, reaction condition algorithm (T, C) relating to the constraint conditions is added to the $\Delta G^{\ddagger}$ selected in the third embodiment, and a reaction time (and a yield) is presented from a general reaction rate equation using Equation (2) described above.

As described above, the reactivity prediction system 40 according to the fourth embodiment includes the reaction condition presentation unit 65 that presents predetermined reaction conditions based on activation free energy predicted by the $\Delta G^{\ddagger}$ prediction unit 63 of the processing unit 44 and the predetermined constraint conditions. The predetermined constraint conditions include at least one of a reaction time, a reaction field temperature, and a concentration of a reactant. According to this configuration, it is possible to present reaction conditions (a reaction field temperature and a concentration of a reactant) that can be implemented within the constraint conditions and that allow a reaction to be completed within a reaction time determined by flow path restrictions, based on the selected $\Delta G^{\ddagger}$ and to display a reaction completion time (prediction time) for reference.

[Fifth embodiment]

In the first to fourth embodiments, the reactivity prediction system 40 is set to predict activation free energy ($\Delta G^{\ddagger}$). In contrast, in the fifth embodiment, the reactivity prediction system 40 is set to predict reaction enthalpy ($\Delta H$). Hereinafter, the fifth embodiment will be described.

<$\Delta H$ Prediction>

In the present embodiment, the generation unit 53 shown in FIG. 3 generates a learned model MD using reaction enthalpy ($\Delta H$) measured by the soft sensor 1 as training data and reaction conditions under which the reaction enthalpy ($\Delta H$) is measured as a first dataset. In addition, the model storage unit 55 shown in FIG. 3 stores the learned model MD generated by the generation unit 53.

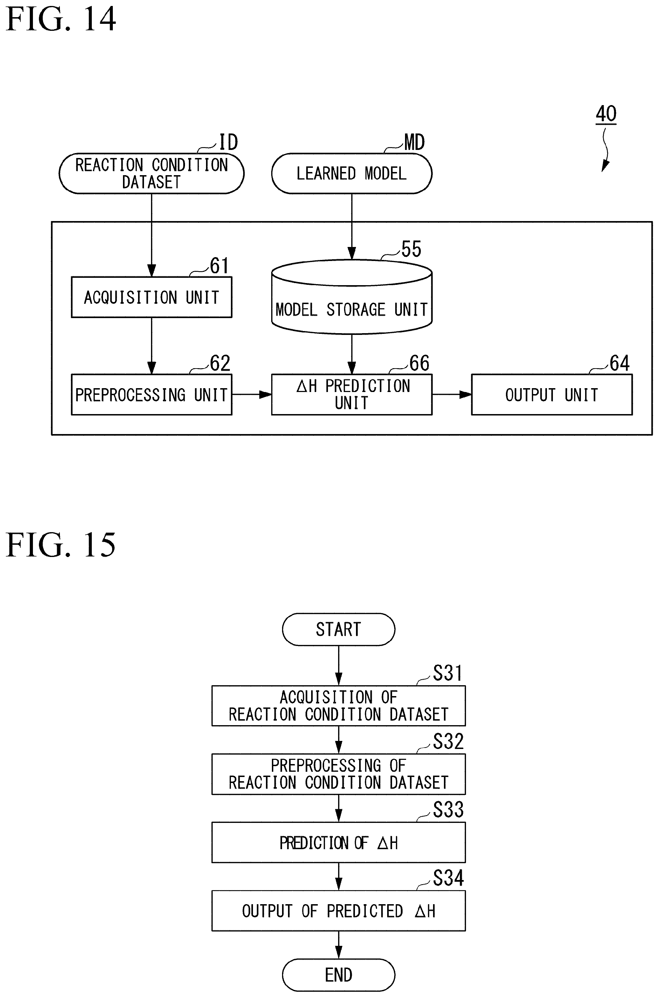

FIG. 14 is a block diagram showing an example of a functional configuration pertaining to $\Delta H$ prediction of the reactivity prediction system 40 according to the fifth embodiment of the present invention. In FIG. 14, the same configuration as that shown in FIG. 6 will be denoted by the same reference numerals. As shown in FIG. 14, the reactivity prediction system 40 includes a $\Delta H$ prediction unit 66. The $\Delta H$ prediction unit 66 is realized by the processing unit 44 shown in FIG. 2. In other words, the processing unit 44 executes a program stored in the storage unit 43 to realize the function of the $\Delta H$ prediction unit 66.

The acquisition unit 61 operates the operation unit 41 of the reactivity prediction system 40 shown in FIG. 2 to acquire input reaction conditions (reaction condition datasets ID), for example. When the reaction condition datasets ID are stored in the storage unit 43, the acquisition unit 61 may acquire the reaction condition datasets ID from the storage unit 43. The reaction condition datasets ID may include at least a reactant, a catalyst, and a solvent.

The preprocessing unit 62 performs preprocessing necessary for predicting $\Delta H$ on the reaction condition datasets ID acquired by the acquisition unit 61. If the preprocessing for the reaction condition datasets ID is not required, the preprocessing unit 62 is not be repeated.

The $\Delta H$ prediction unit 66 performs $\Delta H$ prediction using the learned model MD stored in the model storage unit 55 and the reaction condition datasets ID preprocessed by the preprocessing unit 62. The output unit 64 displays prediction results of the $\Delta H$ prediction unit 66.

FIG. 15 is a flowchart showing an example of $\Delta H$ prediction processing performed by the reactivity prediction system 40 according to the fifth embodiment of the present invention. The processing of the flowchart shown in FIG. 15 is started such that, for example, an operator using the reactivity prediction system 40 operates the operation unit 41 of the reactivity prediction system 40 to give an instruction to start $\Delta H$ prediction processing.

When the processing of the flowchart shown in FIG. 15 begins, first, the acquisition unit 61 of the reactivity prediction system 40 acquires the reaction condition datasets ID to be predicted (Step S31). For example, the acquisition unit 61 acquires the reaction condition datasets ID input from the operation unit 41.

Next, the preprocessing unit 62 of the reactivity prediction system 40 performs preprocessing of the acquired reaction condition datasets ID (Step S32).

Next, the $\Delta H$ prediction unit 66 of the reactivity prediction system 40 performs $\Delta H$ prediction using the learned model MD stored in the model storage unit 55 and the reaction condition datasets ID preprocessed by the preprocessing unit 62 (Step S33).

Subsequently, the output unit 64 of the reactivity prediction system 40 outputs the predicted $\Delta H$ (Step S34).

Accordingly, the predicted ΔH in the ΔH prediction unit 66 is displayed in the output unit 64.

As described above, the reactivity prediction system 40 according to the present embodiment includes the storage unit 43 that stores a learned model MD that has learned a relationship between measured reaction enthalpy (ΔH) and reaction conditions under which the reaction enthalpy is measured; and the processing unit 44 that predicts reaction enthalpy from input reaction conditions (reaction condition datasets ID) using the learned model MD. According to this configuration, since ΔH is predicted using machine learning from actually measured ΔH by the soft sensor 1, a more reliable ΔH than ΔH derived by only a quantum chemical calculation is obtained.

[Sixth embodiment]

<Reactivity prediction system>

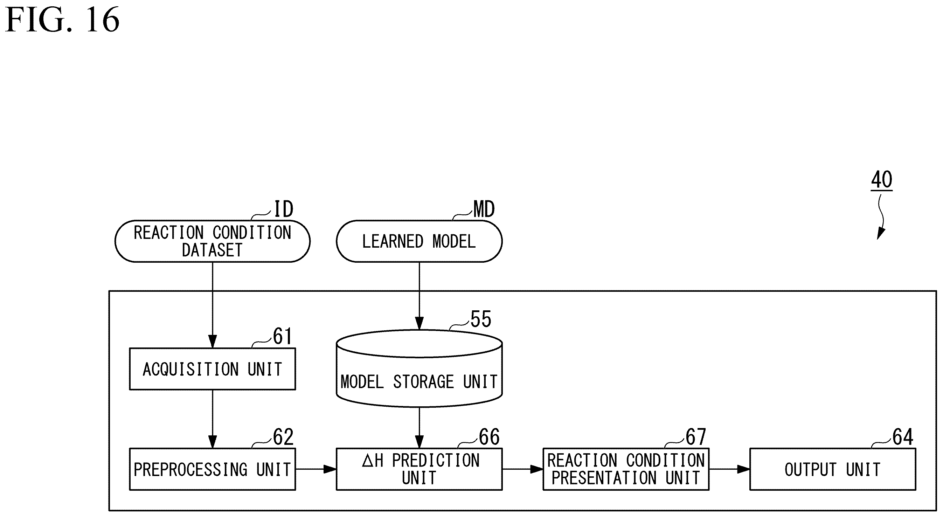

FIG. 16 is a block diagram showing a key configuration of a reactivity prediction system 40 according to a sixth embodiment of the present invention. In FIG. 16, the same configuration as that shown in FIG. 6 will be denoted by the same reference numerals. As shown in FIG. 16, the reactivity prediction system 40 according to the sixth embodiment further includes: a reaction condition presentation unit 67 that presents predetermined reaction conditions based on reaction enthalpy predicted by a ΔH prediction unit 66. The reaction condition presentation unit 67 is realized by the processing unit 44 shown in FIG. 2. In other words, the processing unit 44 executes a program stored in the storage unit 43 to realize the function of the reaction condition presentation unit 67.

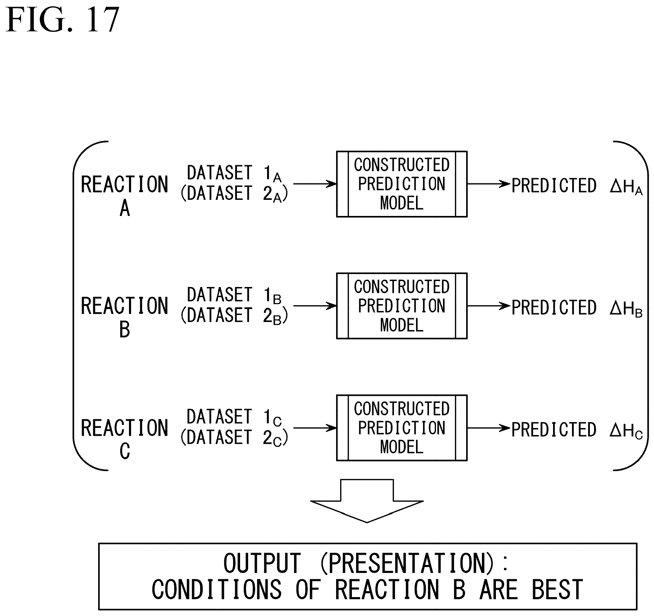

FIG. 17 is an explanatory view showing an example of reaction condition presentation processing performed by a reaction condition presentation unit 67 according to the sixth embodiment of the present invention. The reaction condition presentation processing shown in FIG. 17 presents reaction conditions expected to have the lowest reaction enthalpy among the reaction enthalpy predicted by the ΔH prediction unit 66. For example, the reaction condition presentation unit 67 compares ΔH among reactions A to C and executes an operation to select conditions (combination of reactants) with the lowest value.

Specifically, as shown in FIG. 16, the acquisition unit 61 acquires the reaction conditions (datasets $1_A$ to $1_C$ (datasets $2_A$ to $2_C$) shown in FIG. 17) input for each of the reactions A to C as the above-described reaction condition datasets ID. The datasets $1_A$ to $1_C$ may be reaction conditions including at least a reactant, a catalyst, and a solvent. When inputting the datasets $2_A$ to $2_C$, the datasets $2_A$ to $2_C$ may be feature amounts including at least one of a dipole moment, atomic charge, frequency, and a molecular orbital.

The preprocessing unit 62 performs preprocessing necessary for predicting ΔH on the reaction conditions acquired by the acquisition unit 61. If the preprocessing for the reaction condition datasets ID is not required, the preprocessing unit 62 is not be repeated. The ΔH prediction unit 66 performs ΔH prediction using the learned model MD stored in the model storage unit 55 and the reaction conditions preprocessed by the preprocessing unit 62.

The reaction condition presentation unit 67 presents reaction conditions expected to have the lowest reaction enthalpy among the reaction enthalpy predicted by the ΔH prediction unit 66. In the example of FIG. 17, the reaction condition presentation unit 67 presents the conditions of the reaction B having the lowest ΔH. The reaction condition presentation unit 67 may present ΔH of the reactions A to C as a reference. An output unit 64 displays presentation results of the reaction condition presentation unit 67.

As described above, the reactivity prediction system 40 according to the sixth embodiment includes: the reaction condition presentation unit 67 that presents predetermined reaction conditions based on reaction enthalpy predicted by the ΔH prediction unit 66 of the processing unit 44. The reaction condition presentation unit 67 presents reaction conditions expected to have the lowest reaction enthalpy among the reaction enthalpy predicted by the ΔH prediction unit 66 of the processing unit 44. In this manner, it is possible to present reaction conditions with a small risk in terms of safety and potential side reactions due to reaction heat.

The first to sixth embodiments, the reactivity prediction system 40 is set to predict activation free energy ($\Delta G^{\ddagger}$) or reaction enthalpy (ΔH) as a reaction parameter, but may predict other thermodynamic parameters as reaction parameters.

Although the reactivity prediction system and reactivity prediction method according to the embodiments of the present invention have been described above, the present invention is not limited to the above-described embodiments and can be freely modified within the scope of the present invention. For example, the above-described embodiments may be combined or substituted.

REFERENCE SIGNS LIST

**[0142]**

　　1 Soft sensor (measurement device)
　　2 Simulator (Calculation device)

40 Reactivity prediction system
40A First interface
40B Second interface
43 Storage unit
44 Processing unit
53 Generation unit
63 $\Delta G^{\ddagger}$ Prediction unit
65 Reaction condition presentation unit
66 $\Delta H$ Prediction unit
67 Reaction condition presentation unit
D11 First dataset
D12 Training data
D21 Second dataset
DS Learning dataset
MD Learned model

## Claims

1. A reactivity prediction system comprising:

   a storage unit that stores a learned model that has learned a relationship between measured reaction parameters and reaction conditions under which the reaction parameters are measured; and
   a processing unit that predicts reaction parameters from input reaction conditions using the learned model.

2. The reactivity prediction system according to claim 1, further comprising:
   a generation unit that generates the learned model using the measured reaction parameters as training data and the reaction conditions under which the reaction parameters are measured as first datasets.

3. The reactivity prediction system according to claim 2,
   wherein the reaction conditions of the first datasets include at least one of a reactant, a solvent, a catalyst, an additive, a reagent, a temperature, and a reaction time.

4. The reactivity prediction system according to any one of claims 1 to 3, further comprising:
   a first interface connectable to a measurement device that measures the reaction parameters.

5. The reactivity prediction system according to claim 1,
   wherein the learned model is trained to learn a relationship between a feature amount relating to a reaction obtained by a quantum chemical calculation and calculation conditions under which the quantum chemical calculation is performed, as well as the relationship between the measured reaction parameters and the reaction conditions under which the reaction parameters are measured.

6. The reactivity prediction system according to claim 5, further comprising:
   a generation unit that generates the learned model using the measured reaction parameters as training data, the reaction conditions under which the reaction parameters are measured as first datasets, and the feature amount and the calculation conditions as a second dataset.

7. The reactivity prediction system according to claim 6,
   wherein the feature amount of the second dataset includes at least one of a dipole moment, an atomic charge, a frequency, and a molecular orbital.

8. The reactivity prediction system according to any one of claims 5 to 7, further comprising:
   a second interface connectable to a calculation device that performs the quantum chemical calculation.

9. The reactivity prediction system according to claim 1, further comprising:
   a reaction condition presentation unit that presents predetermined reaction conditions based on the reaction parameters predicted by the processing unit.

**10.** The reactivity prediction system according to claim 9,
wherein the reaction condition presentation unit presents reaction conditions expected to have the lowest reaction parameter among the reaction parameters predicted by the processing unit as the predetermined reaction conditions.

**11.** The reactivity prediction system according to claim 1, further comprising:
a reaction condition presentation unit that presents predetermined reaction conditions based on the reaction parameters predicted by the processing unit and predetermined constraint conditions.

**12.** The reactivity prediction system according to claim 11,
wherein the predetermined constraint conditions include at least one of a reaction time, a reaction field temperature, and a concentration of a reactant.

**13.** The reactivity prediction system according to claim 1,
wherein the reaction parameters are thermodynamic parameters.

**14.** The reactivity prediction system according to claim 13,
wherein the thermodynamic parameters are activation free energy or reaction enthalpy.

**15.** A reactivity prediction method comprising:

a first step of inputting reaction conditions; and
a second step of predicting reaction parameters from reaction conditions input in the first step using a learned model that has learned a relationship between measured reaction parameters and reaction conditions under which the reaction parameters are measured.

FIG. 1

FIG. 2

# FIG. 3

# FIG. 4

FIG. 5

```
                    ┌─────────────┐
                    │    START    │
                    └──────┬──────┘
                           │
                           ▼            ╭─ S11
              ┌───────────────────────┐
              │    ACQUISITION OF     │
              │   LEARNING DATASET    │
              └───────────┬───────────┘
                          │             ╭─ S12
              ┌───────────────────────┐
              │   PREPROCESSING OF    │
              │   LEARNING DATASET    │
              └───────────┬───────────┘
                          │
      ┌───────────────────┤             ╭─ S13
      │       ┌───────────────────────┐
      │       │ PERFORMING OF LEARNING │
      │       └───────────┬───────────┘
      │                   │
      │                   ▼              ╭─ S14
      │         ╱─────────────────────╲
      │  NO    ╱      COMPLETION OF     ╲
      └───────╱         LEARNING?        ╲
              ╲                          ╱
               ╲────────────┬──────────╱
                            │ YES
                            ▼            ╭─ S15
              ┌───────────────────────┐
              │  STORAGE OF LEARNED MODEL │
              └───────────┬───────────┘
                          │
                    ┌─────▼──────┐
                    │     END     │
                    └─────────────┘
```

FIG. 6

# FIG. 7

```
          START
            │
            │       S21
            ▼
    ACQUISITION OF
 REACTION CONDITION DATASET
            │       S22
            ▼
   PREPROCESSING OF
 REACTION CONDITION DATASET
            │       S23
            ▼
   PREDICTION OF  △G‡
            │       S24
            ▼
 OUTPUT OF PREDICTED  △G‡
            │
            ▼
          END
```

Step S23: PREDICTION OF $\Delta G^{\ddagger}$

Step S24: OUTPUT OF PREDICTED $\Delta G^{\ddagger}$

FIG. 8

FIG. 9

EP 4 629 251 A1

# FIG. 10

Block diagram (reference 40):

- REACTION CONDITION DATASET (ID) → ACQUISITION UNIT (61) → PREPROCESSING UNIT (62) → $\Delta G^{\ddagger}$ PREDICTION UNIT (63) → REACTION CONDITION PRESENTATION UNIT (65) → OUTPUT UNIT (64)
- LEARNED MODEL (MD) → MODEL STORAGE UNIT (55) → $\Delta G^{\ddagger}$ PREDICTION UNIT (63)

# FIG. 11

REACTION A $\quad$ DATASET $1_A$ (DATASET $2_A$) $\rightarrow$ CONSTRUCTED PREDICTION MODEL $\rightarrow$ PREDICTED $\Delta G_A^{\ddagger}$ $\rightarrow$ GENERAL REACTION RATE EQUATION $\rightarrow$ REACTION TIME AND YIELD OF A

REACTION B $\quad$ DATASET $1_B$ (DATASET $2_B$) $\rightarrow$ CONSTRUCTED PREDICTION MODEL $\rightarrow$ PREDICTED $\Delta G_B^{\ddagger}$ $\rightarrow$ GENERAL REACTION RATE EQUATION $\rightarrow$ REACTION TIME AND YIELD OF B

REACTION C $\quad$ DATASET $1_C$ (DATASET $2_C$) $\rightarrow$ CONSTRUCTED PREDICTION MODEL $\rightarrow$ PREDICTED $\Delta G_C^{\ddagger}$ $\rightarrow$ GENERAL REACTION RATE EQUATION $\rightarrow$ REACTION TIME AND YIELD OF C

OUTPUT (PRESENTATION):
CONDITIONS OF REACTION B ARE BEST

EP 4 629 251 A1

# FIG. 12

FIG. 13

$\Delta G^{\ddagger}$ SELECTED
BY OPERATION →$\Delta G^{\ddagger}$→ CONDITION PRESENTATION ALGORITHM → REACTION CONDITIONS T, C → GENERAL REACTION RATE EQUATION →REACTION TIME (AND YIELD)

・TEMPERATURE T (UPPER LIMIT, LOWER LIMIT)
・CONCENTRATION C (UPPER LIMIT)

## FIG. 14

## FIG. 15

# FIG. 16

FIG. 17

REACTION A — DATASET $1_A$ (DATASET $2_A$) → CONSTRUCTED PREDICTION MODEL → PREDICTED $\Delta H_A$

REACTION B — DATASET $1_B$ (DATASET $2_B$) → CONSTRUCTED PREDICTION MODEL → PREDICTED $\Delta H_B$

REACTION C — DATASET $1_C$ (DATASET $2_C$) → CONSTRUCTED PREDICTION MODEL → PREDICTED $\Delta H_C$

OUTPUT (PRESENTATION):
CONDITIONS OF REACTION B ARE BEST

## INTERNATIONAL SEARCH REPORT

| International application No. |
|---|
| **PCT/JP2023/041624** |

**A.    CLASSIFICATION OF SUBJECT MATTER**

*G16C 20/70*(2019.01)i
FI:    G16C20/70

According to International Patent Classification (IPC) or to both national classification and IPC

**B.    FIELDS SEARCHED**

Minimum documentation searched (classification system followed by classification symbols)

G16C10/00-99/00; G06Q10/00-99/00; G16Z99/00; G06N3/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | WO 2020/184240 A1 (FUJIFILM CORPORATION) 17 September 2020 (2020-09-17) paragraphs [0009], [0022], [0035], [0040], [0044]-[0047], [0052]-[0074], fig. 1 | 1-4, 9-12, 15 |
| Y | | 13-14 |
| A | | 5-8 |
| Y | JP 2022-102726 A (SHOWA DENKO KK) 07 July 2022 (2022-07-07) paragraphs [0017]-[0019], [0066], [0108] | 13-14 |
| A | | 5-8 |
| A | JP 2020-526751 A (SRI INTERNATIONAL) 31 August 2020 (2020-08-31) paragraphs [0005]-[0008], [0011], [0024], [0026], [0032], [0037]-[0039], [0047], [0050]-[0052] | 1-15 |
| A | JP 2022-27571 A (SAMSUNG ELECTRONICS CO LTD) 10 February 2022 (2022-02-10) paragraphs [0017]-[0018], [0021], [0024]-[0025], [0031]-[0034], [0088]-[0092] | 1-15 |

☑ Further documents are listed in the continuation of Box C.          ☑ See patent family annex.

| | | |
|---|---|---|
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
|---|---|
| **16 January 2024** | **30 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2023/041624** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2003/0078740 A1 (KIEKEN, Laurent) 24 April 2003 (2003-04-24)<br>paragraphs [0038]-[0047], [0055], [0066]-[0068] | 1-15 |
| A | CN 113140260 A (TENCENT TECHNOLGY SHENZHEN CO LTD) 20 July 2021 (2021-07-20)<br>paragraphs [0056]-[0057], [0081]-[0094], [0103], [0108] | 1-15 |

Form PCT/ISA/210 (second sheet) (July 2022)

**INTERNATIONAL SEARCH REPORT**
**Information on patent family members**

| International application No. |
| --- |
| **PCT/JP2023/041624** |

| Patent document cited in search report | | | Publication date (day/month/year) | Patent family member(s) | Publication date (day/month/year) |
| --- | --- | --- | --- | --- | --- |
| WO | 2020/184240 | A1 | 17 September 2020 | US 2021/0390369 A1 paragraphs [0009], [0034], [0047], [0068], [0072]-[0075], [0081]-[0112], fig. 1 EP 3936224 A1 CN 113543874 A | |
| JP | 2022-102726 | A | 07 July 2022 | (Family: none) | |
| JP | 2020-526751 | A | 31 August 2020 | US 2020/0225251 A1 paragraphs [0005]-[0008], [0011], [0034], [0036], [0042], [0047]-[0049], [0057], [0060]-[0062] WO 2019/006391 A1 EP 3645166 A1 CN 111201085 A | |
| JP | 2022-27571 | A | 10 February 2022 | US 2022/0036182 A1 paragraphs [0048]-[0049], [0052], [0055]-[0056], [0061]-[0064], [0120]-[0124] EP 3945526 A1 CN 114093430 A | |
| US | 2003/0078740 | A1 | 24 April 2003 | US 2002/0086791 A1 WO 03/020417 A1 | |
| CN | 113140260 | A | 20 July 2021 | (Family: none) | |

Form PCT/ISA/210 (patent family annex) (July 2022)

**REFERENCES CITED IN THE DESCRIPTION**

**Patent documents cited in the description**

- JP 2008150337 A

- JP 2010009257 A